# FASCICULE DE BREVET EUROPEEN

(11) **EP 1 397 114 B1**
(45) Date de publication et mention de la délivrance du brevet: **11.07.2012**
(21) Numéro de dépôt: 02740848.3
(22) Date de dépôt: 31.05.2002
(51) Int. Cl.: A61K 8/04, A61K 8/25, A61K 8/87, A61K 8/896, A61Q 5/06

(54) **Composition en aerosol comprenant des particules de silicate et des polymères**
Aerosolzusammensetzung, die Silikatpartikel und Polymere enthält
Aerosol composition comprising silicate particles and polymers

(30) Priorité: 31.05.2001 FR 0107156
(43) Date de publication de la demande: 17.03.2004
(73) Titulaire: L'Oréal, 75008 Paris (FR)
(72) Inventeur: BELLI, Emmanuelle, F-92600 Asnieres (FR); PATAUT, Françoise, F-75017 Paris (FR); NOCERINO, Cécile, F-75006 Paris (FR); GILLES, Audrey, F-78140 Velizy (FR)
(74) Mandataire: Bourdeau, Françoise
(86) Numéro de dépôt international: PCT/FR2002/001847
(87) Numéro de publication internationale: WO 2002/096379

(56) Documents cités:
- DE-A- 2 542 338
- US-A- 4 983 377
- US-A- 6 126 929

## Description

La présente invention est relative à une composition conditionnée dans un dispositif aérosol comprenant un agent propulseur et une phase liquide qui contient, dans un milieu cosmétiquement acceptable, des particules de silicate, à un procédé de traitement cosmétique des cheveux et à une utilisation de cette composition en tant que produit de coiffage.

Les produits coiffants sont habituellement utilisés pour construire, structurer la coiffure et lui apporter une tenue durable. Pour cela, des polymères filmogènes sont introduits dans un milieu cosmétiquement acceptable. Cependant, certains polymères entraînent un durcissement de la chevelure et/ou un caractère collant. De ce fait, les cheveux sont souvent collés entre eux, et le démêlage induit une destruction de la forme de la chevelure.

Pour remédier à ces inconvénients, il a déjà été proposé l'utilisation de poudre solide dans les compositions cosmétiques, telles que des poudres d'oxydes métalliques. Par exemple, le brevet US 3 819 827 décrit des produits pour la mise en plis des cheveux comprenant de 0,2 à 6 % en poids de particules d'oxyde d'aluminium présentant une taille de particule d'environ 30 mµ.

De la même manière, la demande de brevet EP 1 005 849 décrit l'utilisation de fines poudres solides inorganiques, comme les poudres de carbonate de calcium, d'hydroxyde d'aluminium, de carbonate de magnésium, de phosphate de calcium, d'oxalàte de calcium ou de sulfate de baryum, dans des compositions cosmétiques capillaires contenant au moins deux phases liquides. Il est précisé que de telles compositions procurent sur les cheveux des effets spécialement avantageux lorsque la composition est conditionnée, sans gaz propulseur, dans un dispositif susceptible de pulvériser la composition sur les cheveux.

Le brevet DE 25 42 338 décrit des lotions et des sprays, sans propulseur, pour la fixation des cheveux comprenant des particules de silicates, dans un milieu alcoolique ou hydroalcoolique, la concentration en silicates étant comprise entre 0,1 et 4%.

Le brevet US-A-4 983 377 décrit une composition cosmétique capillaire conditionnée dans un dispositif aérosol contenant un agent propulseur et une phase liquide comprenant: (i) des particules solides contenant au moins 10% en poids d'un silicate et (ii) un polymère fixant; les particules solides utilisées ne contiennent pas d'aluminium. On utilise des solvants volatils, l'eau pouvant être utilisée pour remplacer en partie le solvant volatil.

La Demanderesse a trouvé, de manière surprenante et inattendue, qu'en choisissant, comme particules solides, des particules de silicate, il est possible d'obtenir de bonnes propriétés cosmétiques et d'obtenir un coiffage sans coller ni surcharger les cheveux, dans la mesure où la composition est conditionnée dans un dispositif particulier, à savoir un dispositif aérosol, quand la composition cosmétique comprend, en outre au moins un polymère choisi parmi les polymères fixants, les polymères épaississants ou un mélange de ceux-ci.

L'invention a pour objet une composition cosmétique, notamment capillaire, conditionnée dans un dispositif aérosol, contenant un agent propulseur et une phase liquide qui comprend, dans un milieu cosmétiquement acceptable :
(i) des particules solides contenant au moins 10 % en poids d'au moins un silicate,
(ii) au moins un polymère choisi parmi les polymères fixants, les polymères épaississants ou un mélange de ceux-ci ; et
(iii) de l'eau, dans une quantité comprise entre 15 et 45% en poids par rapport au poids total de la composition, lesdites particules solides contenant moins de 1 % en poids d'aluminium.

Un autre objet de la présente invention consiste en un procédé de traitement cosmétique des cheveux mettant en oeuvre la composition selon l'invention.

L'invention a encore pour objet l'utilisation de la composition en cosmétique capillaire, notamment pour fixer et/ou maintenir les cheveux dans une forme souhaitée.

D'autres objets, caractéristiques, aspects et avantages de l'invention apparaîtront encore plus clairement à la lecture de la description et des divers exemples qui suivent

### PARTICULES

De préférence, les particules solides contiennent entre 0 et 1 % d'aluminium, et plus préférentiellement encore entre 0 et 0,5 %.

Avantageusement, les particules solides présentent une taille primaire moyenne en nombre comprise entre 2 nm et 1 µm.

Les particules contenant au moins 10 % en poids d'au moins un silicate présentent, de préférence, une taille primaire moyenne en nombre comprise entre 5 et 500 nm, et plus préférentiellement entre 10 et 250 nm.

Les particules selon l'invention peuvent avoir une forme quelconque, par exemple la forme de sphères, de paillettes, d'aiguilles, de plaquettes ou des formes totalement aléatoires.

De préférence, il s'agit de silicates de sodium, de magnésium et/ou de lithium, comme les composés commercialisés par la Société Laporte sous les dénominations LAPONITE XLG et LAPONITE XLS.

Au sens de la présente invention, on entend par *"taille primaire de particule",* la dimension maximale qu'il est possible de mesurer entre deux points diamétralement opposés d'une particule individuelle. La taille peut être déterminée, par exemple, par microscopie électronique à transmission ou à partir de la mesure de la surface spécifique par la méthode BET ou bien par l'intermédiaire d'un granulomètre laser.

Dans le cas où les particules sont formées par du silicate et d'autres charges, le silicate se trouve à l'état libre et ne forme pas de liaisons chimiques avec les autres charges. Il s'agit alors d'un alliage entre le silicate et d'autres charges, notamment avec des oxydes de métaux ou de métalloïdes, en particulier obtenu par fusion thermique de ces différents constituants.

Lorsque les particules contenant au moins 10 % en poids d'au moins un silicate comprennent, en outre, un oxyde de métal ou de métalloïde, celui-ci est notamment choisi parmi l'oxyde de silicium, de bore, d'aluminium.

De préférence, les particules contiennent au moins 50 % en poids de silicate, mieux encore au moins 70 % en poids, et les particules constituées à plus de 90 % en poids de silicate sont particulièrement préférées selon la présente invention.

Le silicate convenant dans les compositions de la présente invention peut être d'origine naturelle ou peut être d'origine synthétique.

Les particules contenant du silicate selon l'invention sont, notamment, utilisées en une quantité comprise entre 0,01 % et 10 % en poids, et de préférence entre 0,5 % et 3 % en poids par rapport au poids total de la composition.

La composition selon l'invention peut également contenir d'autres types de particules, par exemple, des particules d'oxyde de titane ou de zinc, d'aluminium.

### POLYMERES FIXANTS

Le polymère fixant (b) est choisi parmi les polymères fixants cationiques, anionique, amphotères, non ioniques et leurs mélanges. Un polymère fixant est un polymère capable de maintenir et/ou de fixer la forme dé la coiffure.

Ces polymères fixants peuvent être utilisés sous forme solubilisée ou encore sous forme de dispersion de particules solides de polymère.

Les polymères fixants (b) cationiques, anioniques, amphotères et non ioniques utilisables conformément à l'invention sont décrits ci-après.

Les polymères fixants anioniques généralement utilisés sont des polymères comportant des groupements dérivés d'acide carboxylique, sulfonique ou phosphorique et ont un poids moléculaire compris entre environ 500 et 5.000.000.

1) Les groupements carboxyliques sont apportés par des monomères mono ou diacides carboxyliques insaturés tels que ceux répondant à la formule : 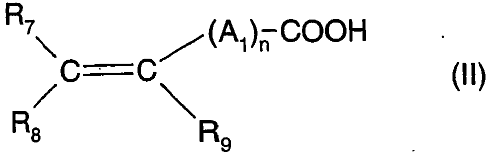 dans laquelle n est un nombre entier de 0 à 10, A₁ désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle ;

Dans la formule précitée un radical alkyle inférieur désigne de préférence un groupement ayant 1 à 4 atomes de carbone et en particulier, méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL E ou K par la société ALLIED COLLOID et ULTRAHOLD par la société BASF. Les copolymères d'acide acrylique et d'acrylamide vendus sous la forme de leur sel de sodium sous les dénominations RETEN 421, 423 ou 425 par la Société HERCULES, les sels de sodium des acides polyhydroxycarboxyliques.
B) Les copolymères des acides acrylique ou méthacrylique avec un monomère monoéthylénique tel que l'éthylène, le styrène, les esters vinyliques, les esters d'acide acrylique ou méthacrylique, éventuellement greffés sur un polyalkylène glycol tel que le polyéthylène glycol et éventuellement réticulés. De tels polymères sont décrits en particulier dans le brevet français 1.222.944 et la demande allemande 2.330.956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C₁-C₂₀ par exemple de lauryle tel que celui vendu par la société ISP sous la dénomination ACRYLIDONE LM et les terpolymères acide méthacrylique/ acrylate d'éthyle/ acrylate de tertiobutyle tel que le produit vendu sous la dénomination LUVIMER 100 P par la société BASF.
C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ou encore un ester vinylique, allylique ou méthallylique d'un acide carboxylique α- ou β-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 vendues par la société NATIONAL STARCH.
D) les copolymères dérivés d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US 2.047.398, 2.723.248, 2.102.113, le brevet GB 839.805 et notamment ceux vendus sous les dénominations GANTREZ AN ou ES par la société ISP.
   - les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
   les fonctions anhydrides de ces copolymères étant éventuellement monoestérifiées ou monoamidifiées.
   Ces polymères sont par exemple décrits dans les brevets français 2.350.384 et 2.357.241 de la demanderesse.
E) les polyacrylamides comportant des groupements carboxylates.

Les polymères comprenant les groupements sulfoniques sont des polymères comportant des motifs vinylsulfonique, styrène sulfonique, naphtalène sulfonique ou acrylamido alkylsulfonique.

Ces polymères peuvent être notamment choisis parmi :
- les sels de l'acide polyvinylsulfonique ayant un poids moléculaire compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone.
- les sels de l'acide polystyrène sulfonique les sels de sodium ayant un poids moléculaire d'environ 500.000 et d'environ 100.000 vendus respectivement sous les dénominations Flexan 500 et Flexan 130 par National Starch. Ces composés sont décrits dans le brevet FR 2.198.719.
- les sels d'acides polyacrylamide sulfoniques ceux mentionnés dans le brevet US 4.128.631 et plus particulièrement l'acide polyacrylamidoéthylpropane sulfonique vendu sous la dénomination COSMEDIA POLYMER HSP 1180 par Henkel.

Selon l'invention, les polymères fixants anioniques sont de préférence choisis parmi les copolymères d'acide acrylique tels que le terpolymère acide acrylique / acrylate d'éthyle / N-tertiobutylacrylamide vendu sous la dénomination ULTRAHOLD STRONG par la société BASF, les copolymères dérivés d'acide crotonique tels que les terpolymères acétate de vinyle / tertio-butyl benzoate de vinyle / acide crotonique et les terpolymères acide crotonique / acétate de vinyle/néododécanoate de vinyle vendus sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que le copolymère méthylvinyléther/anhydride maléique mono estérifié vendu sous la dénomination GANTREZ ES 425 par la société ISP, les copolymères d'acide méthacrylique et de méthacrylate de méthyle vendus sous la dénomination EUDRAGIT L par la société ROHM PHARMA, le copolymère d'acide méthacrylique et d'acrylate d'éthyle vendu sous la dénomination LUVIMER MAEX ou MAE par la société BASF, le copolymère acétate de vinyle/acide crotonique vendu sous la dénomination LUVISET CA 66 par la société BASF et le copolymère acétate de vinyle/acide crotonique greffé par du polyéthylèneglycol sous la dénomination ARISTOFLEX A par la société BASF.

Les polymères fixants anioniques les plus particulièrement préférés sont choisis parmi le copolymère méthylvinyléther / anhydride maléique mono estérifié vendu sous la dénomination GANTREZ ES 425 par la société ISP, le terpolymère acide acrylique / acrylate d'éthyle / N-tertiobutylacrylamide vendu sous la dénomination ULTRAHOLD STRONG par la société BASF, les copolymères d'acide méthacrylique et de méthacrylate de méthyle vendus sous la dénomination EUDRAGIT L par la société ROHM PHARMA, les terpolymères acétate de vinyle / tertio-butyl benzoate de vinyle / acide crotonique et les terpolymères acide crotonique / acétate de vinyle / néododécanoate de vinyle vendus sous la dénomination Résine 28-29-30 par la société NATIONAL STARCH, le copolymère d'acide méthacrylique et d'acrylate d'éthyle vendu sous la dénomination LUVIMER MAEX OU MAE par la société BASF, le terpolymère de vinylpyrrolidone / acide acrylique/méthacrylate de lauryle vendu sous la dénomination ACRYLIDONE LM par la société ISP.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques ou bien B et C peuvent désigner des groupements dérivant de monomères zwittérioniques de carboxybétaïnes ou de sulfobétaïnes;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné ou bien B et C font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères fixants amphotères répondant à la définition donnée ci-dessus plus particulièrement préférés sont choisis parmi les polymères suivants :
1) les polymères résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl acrylamide, le N-tertiooctyl acrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléïque, fumarique ainsi que les monoesters d'alkyle ayant 1 à 4 atomes de carbone des acides ou des anhydrides maléïque ou fumarique.

Les comonomères basiques préférés sont des méthacrylates d'aminoéthyle, de butyl aminoéthyle, de N,N'-diméthylaminoéthyle, de N-tertio-butylaminoéthyle.

On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) les polyamino amides réticulés et alcoylés partiellement ou totalement dérivant de polyaminoamides de formule générale III: 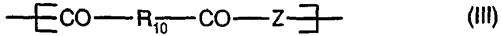 dans laquelle R₁₀ représente un radical divalent dérivé d'un acide dicarboxylique saturé, d'un acide aliphatique mono ou dicarboxylique à double liaison éthylénique, d'un ester d'un alcanol inférieur ayant 1 à 6 atome de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical IV 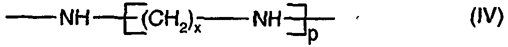 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (IV) ci-dessus, dans lequel x=2 et p=1 1 et qui dérive de l'éthylènediamine, ou le radical dérivant de la pipérazine 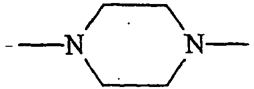
   c) dans les proportions de 0 à 20 moles % le radical -NH-(CH₂)₆-NH-dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.

Les alcanes sultones utilisées dans l'alcoylation sont de préférence la propane ou la butane sultone, les sels des agents d'alcoylation sont de préférence les sels de sodium ou de potassium.
(4) les polymères comportant des motifs zwittérioniques de formule V: 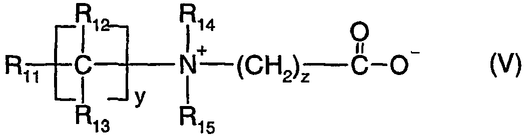 dans laquelle R₁₁ désigne un groupement insaturé polymérisable tel qu'un groupement acrylate, méthacrylate, acrylamide ou méthacrylamide, y et z représente un nombre entier de 1 à 3, R₁₂ et R₁₃ représentent un atome d'hydrogène, méthyle, éthyle ou propyle, R₁₄ et R₁₅ représentent un atome d'hydrogène ou un radical alkyle de telle façon que la somme des atomes de carbone dans R₁₄ et R₁₅ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle / méthacrylate de diméthyl carboxyméthylammonio éthyl tel que le produit vendu sous la dénomination DIAFORMER Z301 par la société SANDOZ.
(5) les polymères dérivés du chitosane comportant des motifs monomères répondant aux formules suivantes : 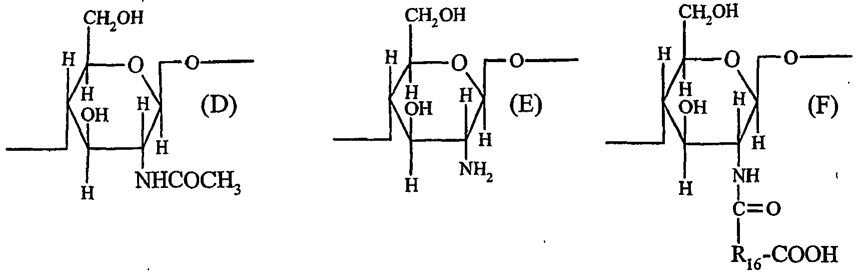 le motif D étant présent dans des proportions comprises entre 0 et 30%, le motif E dans des proportions comprises entre 5 et 50% et le motif F dans des proportions comprises entre 30 et 90%, étant entendu que dans ce motif F, R₁₆ représente un radical de formule : 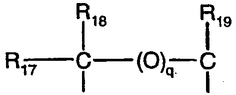 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcolylthio, sulfonique, un reste alcoylthio dont le groupe alcoyle porte un reste amino, l'un au moins des radicaux R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (VI) par exemple décrits dans le brevet français 1 400 366 : 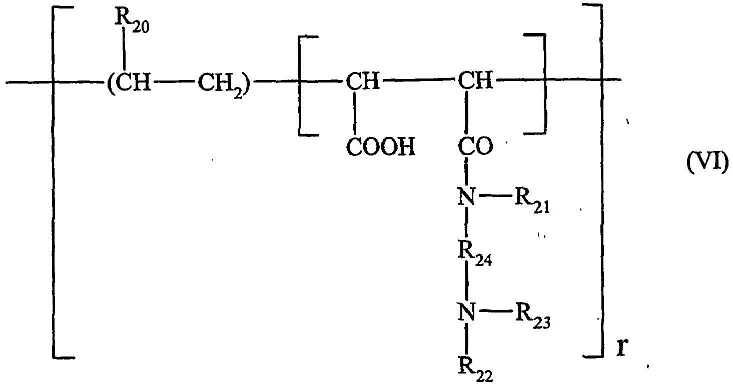 dans laquelle R₂₀ représente un atome d'hydrogène, un radical CH₃O, CH₃CH₂O, phényle, R₂₁ désigne l'hydrogène ou un radical alkyle inférieur tel que méthyle, éthyle, R₂₂ désigne l'hydrogène ou un radical alkyle inférieur tel que méthyle, éthyle, R₂₃ désigne un radical alkyle inférieur tel que méthyle, éthyle ou un radical répondant à la formule : - R₂₄-N(R₂₂)₂, R₂₄ représentant un groupement -CH₂-CH₂- , -CH₂-CH₂-CH₂- , -CH₂-CH(CH₃)-, R₂₂ ayant les significations mentionnées ci-dessus,
   ainsi que les homologues supérieurs de ces radicaux et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (VII)

      où D désigne un radical 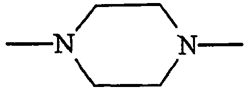 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne.
   b) Les polymères de formule :

      -D-X-D-X- (VII')

      où D désigne un radical 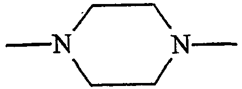 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) les copolymères alkyl(C1-C5)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères fixants amphotères particulièrement préférés selon l'invention sont ceux de la famille (3) tels que les copolymères dont la dénomination CTFA est Octylacrylamide/acrylates/butylaminoethylmethacrylate copolymer tels que les produits vendus sous les dénominations AMPHOMER, AMHOMER LV 71 ou LOVOCRYL 47 par la société NATIONAL STARCH et ceux de la famille (4) tels que les copolymère de méthacrylate de butyle / méthacrylate de diméthyl carboxyméthylammonio- éthyl par exemple vendu sous la dénomination DIAFORMER Z301 par la société SANDOZ.

Les polymères fixants non ioniques utilisables selon la présente invention sont choisis par exemple parmi :
- les homopolymères ou copolymères de vinylpyrrolidone ou de vinylcaprolactame;
- les copolymères de vinylpyrrolidone et d'acétate de vinyle ;
- les polyalkyloxazolines telles que les polyéthyloxazolines proposées par la société DOW CHEMICAL sous les dénominations PEOX 50 000, PEOX 200 000 et PEOX 500 000 ;
- les homopolymères d'acétate de vinyle tels que le produit proposé sous le nom de APPRETAN EM par la société HOECHST ou le produit proposé sous le nom de RHODOPAS A 012 par la société RHONE POULENC ;
- les copolymères d'acétate de vinyle et d'ester acrylique tels que le produit proposé sous le nom de RHODOPAS AD 310 de RHONE POULENC ;
- les copolymères d'acétate de vinyle et d'éthylène tels que le produit proposé sous le nom de APPRETAN TV par la société HOECHST ;
- les copolymères d'acétate de vinyle et d'ester maléïque par exemple de maléate de dibutyle tels que le produit proposé sous le nom de APPRETAN MB EXTRA par la société HOECHST ;
- les copolymères de polyéthylène et d'anhydride maléïque ;
- les homopolymères d'acrylates d'alkyle et les homopolymères de méthacrylates d'alkyle tels que le produit proposé sous la dénomination MICROPEARL RQ 750 par la société MATSUMOTO ou le produit proposé sous la dénomination LUHYDRAN A 848 S par la société BASF ;
- les copolymères d'esters acryliques tels que par exemple les copolymères d'acrylates d'alkyle et de méthacrylates d'alkyles tels que les produits proposés par la société ROHM&HAAS sous les dénominations PRIMAL AC-261 K et EUDRAGIT NE 30 D, par la société BASF sous les dénominations ACRONAL 601, LUHYDRAN LR 8833 ou 8845, par la société HOECHST sous les dénominations APPRETAN N 9213 ou N9212;
- les copolymères d'acrylonitrile et d'un monomère non ionique choisi par exemple parmi le butadiène et les (méth)acrylates d'alkyle ; on peut citer les produits proposés sous les dénominations NIPOL LX 531 B par la société NIPPON ZEON ou ceux proposés sous la dénomination CJ 0601 B par la société ROHM & HAAS ;
- les polyuréthannes tels que les produits proposés sous les dénominations ACRYSOL RM 1020 ou ACRYSOL RM 2020 par la société ROHM & HAAS, les produits URAFLEX XP 401 UZ, URAFLEX XP 402 UZ par la société DSM RESINS ;
- les copolymères d'acrylate d'alkyle et d'uréthanne tels que le produit 8538-33 par la société NATIONAL STARCH ;
- les polyamides tels que le produit ESTAPOR LO 11 proposé par la société RHONE POULENC.

Les radicaux alkyle des polymères non ioniques ont de 1 à 6 atomes de carbone sauf mention contraire.

Les polymères non ioniques qui conviennent tout particulièrement pour la réalisation des compositions conformes à l'invention sont ceux choisis parmi:
* les copolymères de vinyllactame tels que les copolymères de vinylpyrrolidone et d'acétate de vinyle et les copolymères vinylpyrrolidone/ acétate de vinyle/ propionate de vinyle
* la polyvinylcaprolactame LUVISKOL PLUS (BASF)
* les homopolymères d'acétate de vinyle tels que APPRETAN EM (HOECHST) ou RHODOPAS A 012 (RHONE POULENC)
* les polyalkyloxazolines tels que PEOX 50 000 et PEOX 500 000 (DOW CHEMICAL)
* les copolymères d'acétate de vinyle et d'ester acrylique tels que le RHODOPAS AD 310 (RHONE POULENC)
* les copolymères d'acétate de vinyle et d'éthylène tels que APPRETAN TV (HOECHST)
* les copolymères d'acétate de vinyle et d'ester maléique tels que APPRETAN MB EXTRA (HOECHST)
* les homopolymères d'acrylates d'alkyle et les homopolymères de métacrylates d'alkyle tels que LUHYDRAN A 848 S (BASF)
* les copolymères d'esters acryliques tels que PRIMAL AC-261 K (ROHM & HAAS), ACRONAL 601 (BASF) ou APPRETAN N 9213 (HOECHST)
* les copolymères d'acrylonitrile et d'un monomère non-ionique tels que CJ 0601 B (ROHM & HAAS)
* les polyuréthanes tels que ACRYSOL RM 1020 ou ACRYSOL RM 2020 (ROHM & HAAS)
* les copolymères d'acrylate d'alkyle et d'uréthane tels que 8538-33 (NATIONAL STARCH)
* les polyamides tels que ESTAPOR LO 11 (RHONE POULENC)

Selon l'invention, on peut également utiliser les polymères fixants de type siliconés greffés comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037. Ces polymères sont de préférence anioniques ou non ioniques.

De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 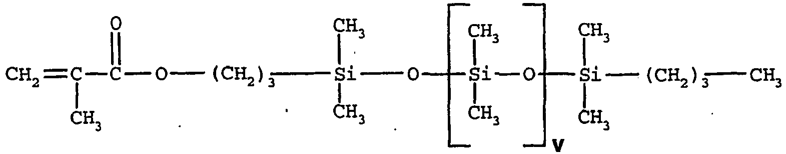 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

On peut aussi utiliser, comme polymères fixants, des polyuréthannes fonctionnalisés ou non, siliconés ou non. A titre de polyuréthanne utilisable dans le cadre de l'invention, on peut citer le produit commercialisé sous l'appellation LUVISET PUR par BASF.

Les polyuréthannes particulièrement visés par la présente invention sont ceux décrits dans les brevets EP 0 751 162, EP 0 637 600, FR 2 743 297 et EP 0 648 485 dont la Demanderesse est Titulaire, ainsi que le brevets EP 0 656 021 ou WO 94/03510 de la Société BASF et EP 0 619 111 de la Société National Starch.

De préférence, on choisit, comme polymères fixants, des polymères anioniques ou non ioniques.

Parmi les polymères cationiques, on peut citer plus particulièrement les polymères du type polyamine, polyaminoamide et polyammonium quaternaire. Ce sont des produits connus.

Les polymères du type polyamine, polyaminoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) les homopolymères ou copolymères dérivés d'esters ou d'amides acryliques ou méthacryliques et comportant au moins un des motifs de formules suivantes: 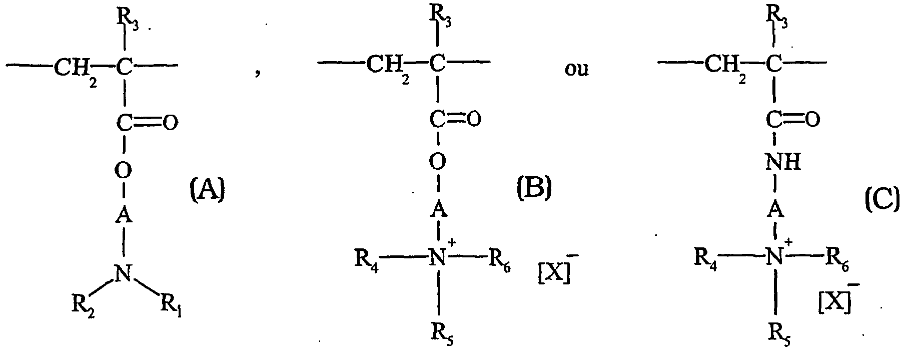 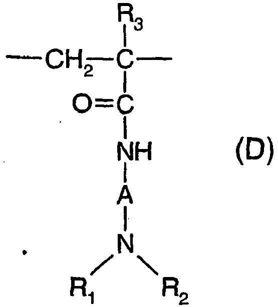 dans lesquelles:
   R₃ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1. à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel que un anion méthosulfate ou un halogénure tel que chlorure ou bromure.

Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.

Ainsi, parmi ces copolymères de la famille (1), on peut citer :
- les copolymères d'acrylamide et de diméthylaminoéthyl méthacrylate quaternisé au sulfate de diméthyle ou avec un hologénure de diméthyle tels que celui vendu sous la dénomination HERCOFLOC par la société HERCULES,
- les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
- le copolymère d'acrylamide et de méthosulfate de méthacryloyloxyéthyltriméthylammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/vinylpyrrolidone tel que le produit vendu sous la dénomination GAFFIX VC 713 par la société ISP,
- les copolymère vinylpyrrolidone / méthacrylamidopropyl dimethylamine commercialisés notamment sous la dénomination STYLEZE CC 10 par ISP.
- et les copolymères vinylpyrrolidone / méthacrylamide de diméthylaminopropyle quaternisé tel que le produit vendu sous la dénomination "GAFQUAT HS 100" par la société ISP.

(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.
   Les produits commercialisés répondant à cette définition sont plus particulièrement les produits vendus sous la dénomination "Celquat L 200" et "Celquat H 100" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.540 et 2.368.508 ;
(7) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d' épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) les cyclopolymères d'alkyl diallyl amine ou de dialkyl diallyl ammonium tels que les homopolymères ou copolymères comportant comme constituant principal de la chaîne des motifs répondant aux formules (VI) ou (VI') : 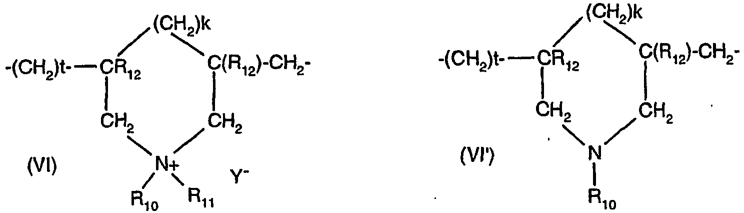 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faibles masses moléculaires moyenne en poids) et les copolymères de chlorure de diallyldiméthylammonium et d'acrylamide commercialisés sous la dénomination "MERQUAT 550".
(10) le polymère de diammonium quaternaire contenant des motifs récurrents répondant à la formule : 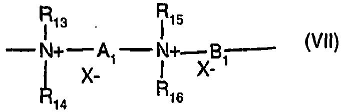 formule (VII) dans laquelle :
   R13, R14, R15 et R16, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont rattachés des hétérocycles contenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne :
   a) un reste de glycol de formule : -O-Z-O-, où Z désigne un radical hydrocarboné linéaire ou ramifié ou un groupement répondant à l'une des formules suivantes :

      -(CH2-CH2-O)x-CH2-CH2-

      -[CH2-CH(CH3)-O]y-CH2-CH(CH3)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH2-CH2-S-S-CH2-CH2- ;
   d) un groupement uréylène de formule : -NH-CO-NH- ;

De préférence, X⁻ est un anion tel que le chlorure ou le bromure.

Ces polymères ont une masse moléculaire moyenne en nombre généralement comprise entre 1000 et 100000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule : 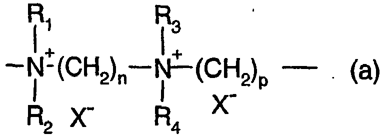 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

Un composé de formule (a) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).
(11) les polymères de polyammonium quaternaires constitués de motifs de formule (VIII): 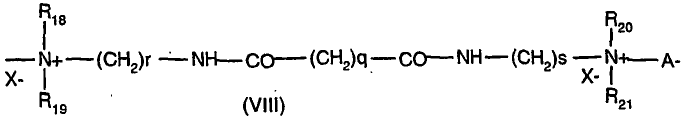 formule dans laquelle :
   R18, R19, R20 et R21, identiques ou différents, représentent un atome d'hydrogène ou un radical méthyle, éthyle, propyle, β-hydroxyéthyle, β-hydroxypropyle ou - CH2CH2(OCH2CH2)pOH,
   où p est égal à 0 ou à un nombre entier compris entre 1 et 6, sous réserve que R18, R19, R20 et R21 ne représentent pas simultanément un atome d'hydrogène,
   r et s, identiques ou différents, sont des nombres entiers compris entre 1 et 6,
   q est égal à 0 ou à un nombre entier compris entre 1 et 34,
   X désigne un atome d'halogène,
   A désigne un radical d'un dihalogénure ou représente de préférence -CH2-CH2-O-CH2-CH2-.

De tels composés sont notamment décrits dans la demande de brevet EP-A-122 324.

On peut par exemple citer parmi ceux-ci, les produits "Mirapol A 15", "Mirapol AD1", "Mirapol AZ1" et "Mirapol 175" vendus par la société Miranol.
(12) Les polymères quaternaires de vinylpyrrolidone et de vinylimidazole tels que par exemple les produits commercialisés sous les dénominations Luviquat FC 905, FC 550 et FC 370 par la société B.A.S.F.
(13) Les polyamines comme le Polyquart H vendu par HENKEL, référencé sous le nom de «POLYETHYLENEGLYCOL (15) TALLOW POLYAMINE» dans le dictionnaire CTFA.
(14) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de «SALCARE^{®} SC 92 » par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE^{®} SC 95 » et « SALCARE^{®} SC 96 » par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les dérivés d'éther de cellulose quaternaires tels que les produits vendus sous la dénomination «JR 400 » par la Société UNION CARBIDE CORPORATION, les cyclopolymères, en particulier les polymères ou copolymères de chlorure de diméthyldiallylammonium et d'acrylamide, vendus sous les dénominations «MERQUAT 100 », « MERQUAT 550 » et « MERQUAT S » par la Société CALGON, les polysaccharides cationiques tels que les gommes de guar modifiées par du chlorure de 2,3-époxypropyl triméthylammonium commercialisées par exemple sous la dénomination « JAGUAR C13S » par la société MEYHALL et les copolymère vinylpyrrolidone / méthacrylamidopropyl dimethylamine commercialisés notamment sous la dénomination STYLEZE CC 10 par la société ISP et leurs mélanges.

On peut aussi utiliser, comme polymères fixants, des polyuréthannes fonctionnalisés ou non, siliconés ou non. A titre de polyuréthanne utilisable dans le cadre de l'invention, on peut citer les produits commercialisés sous l'appellation LUVISET PUR et LUVISET Si PUR par BASF.

### EPAISSISSANT

Au sens de la présente invention, on entend par *«épaississant»,* tout composé qui, à 1 % en poids dans l'eau à la température de 25°C, conduit à une viscosité mesurée au moyen du Rhéomat RM180 supérieure à 0,1 Pa.s, et plus avantageusement encore à 0,2 Pa.s, à un taux de cisaillement de 200 s⁻¹.

L'invention englobe les épaississants naturels et synthétiques. On peut citer comme épaississant naturels les celluloses et leurs dérivés, les gommes de xanthane, de scléroglucane, de gellane, de rhamsan, les alginates, la maltodextrine, l'amidon et ses dérivés, la gomme de karaya, la farine de caroube et les gommes de guar.

Les agents épaississants synthétiques selon l'invention sont avantageusement des polymères ou copolymères d'acide acrylique et/ou méthacrylique, comme les copolymères acide acrylique/acrylate d'éthyle et les polymères carboxyvinyliques. Des exemples de tels polymères ou copolymères sont notamment les "carbomer" (CTFA) vendus par la société GOODRICH sous la dénomination Carbopol ou le polyglycérylméthacrylate commercialisé par la société GUARDIAN sous la dénomination Lubragel ou encore le polyglycérylacrylate commercialisé sous la dénomination Hispagel par la société HISPANO CHIMICA.

Comme autres composés acryliques, on peut citer les copolymères d'acide acrylique ou méthacrylique comprenant au moins un motif acrylate d'alkyle en C1 à C30 et/ou un motif uréthanne éventuellement substitué par une chaîne grasse. On peut en particulier citer le Pemulen TR1 (Goodrich), le Viscophobe DB 1000 (Union Carbide).

On peut encore utiliser comme agent épaississant les polyéthylèneglycols (PEG) et leurs dérivés.

On peut également utiliser avantageusement, en tant qu'agent épaissisant, des polyacrylamides épaississants. Ceux-ci peuvent plus particulièrement être choisis parmi:
- les homopolymères de 2-acrylamido-2-méthylpropane sulfonique réticulés;
- les copolymères d'acrylamide et d'acrylate d'ammonium éventuellement réticulés,
- les copolymères d'acrylamide (ou de méthacrylamide) et de chlorure de méthacryloyloxyéthyl triméthylammonium éventuellement réticulés,
- les copolymères d'acrylamide et d'acide 2-acrylamido 2-méthyl propanesulfonique partiellement ou totalement neutralisés éventuellement réticulés.

Comme copolymères réticulés d'acrylamide / acrylate d'ammonium, utilisés conformément à la présente invention, on peut plus particulièrement citer les copolymères acrylamide / acrylate d'ammonium (5/95 en poids) réticulé par un agent de réticulation à polyinsaturation oléfinique, tel que le divinylbenzène, le tétraallyloxyéthane, le méthylène bis-acrylamide, l'éther diallylique, dès éthers polyallylpolyglycéryliques ou les éthers allyliques d'alcool de la série des sucres, tels que l'érythritol, le pentaérythritol, l'arabitol, le mannitol, le sorbitol ou le glucose.

Des copolymères analogues sont décrits et préparés dans le brevet français FR-2.416.723 et les brevets US-2.798.053 et US-2.923.692.

On utilise en particulier ce type de copolymère réticulé sous forme d'émulsion eau-dans-huile constituée d'environ 30 % en poids dudit copolymère, 25 % en poids d'huile de paraffine, 4 % en poids de mélange de stéarate de sorbitan et d'un dérivé éthoxylé hydrophile et 41 % en poids d'eau. Une telle émulsion est commercialisée sous la dénomination "BOZEPOL C" par la Société HOECHST.

Les copolymères d'acrylamide et de l'acide 2-acrylamido 2-méthyl propane sulfonique, utilisés conformément à la présente invention, sont des copolymères réticulés par un composé à polyinsaturation oléfinique, tels que ceux évoqués précédemment, et partiellement ou totalement neutralisés par un agent de neutralisation tel que la soude, la potasse, l'ammoniaque ou une amine telle que la triéthanolamine ou la monoéthanolamine.

Ils peuvent être préparés en copolymérisant l'acrylamide et le 2-acrylamido 2-méthylpropane sulfonate de sodium par voie radicalaire au moyen d'agents initiateurs du type azobisisobutyronitrile et par précipitation dans un alcool tel que le tertiobutanol.

On utilise plus particulièrement des copolymères obtenus par copolymérisation de 70 à 55 % en moles d'acrylamide et de 30 à 45 % en moles de 2-acrylamido 2-méthylpropane sulfonate de sodium. L'agent de réticulation étant utilisé à des concentrations de 10⁻⁴ à 4.10⁻⁴ mole par mole du mélange de monomères.

Ces copolymères particuliers sont incorporés dans les compositions de l'invention, de façon préférentielle, sous forme d'émulsions eau dans l'huile contenant de 35 à 40 % en poids de ce copolymère, de 15 à 25 % en poids d'un mélange d'hydrocarbures isoparaffiniques en C₁₂-C₁₃, de 3 à 8 % en poids de lauryléther de polyéthylèneglycol à 7 moles d'oxyde d'éthylène et d'eau. Une telle émulsion est commercialisée sous le nom de "SEPIGEL 305" par la société SEPPIC.

Le copolymère d'acrylamide et de chlorure de méthacryloyl oxyéthyl triméthylammonium réticulé, utilisé selon l'invention, est plus particulièrement un copolymère obtenu par copolymérisation de l'acrylamide et du diméthylaminoéthylméthacrylate quaternisé par le chlorure de méthyle, suivie d'une réticulation par un composé à insaturation oléfinique, en particulier le méthylène-bis acrylamide.

On utilise plus particulièrement un copolymère réticulé acrylamide/chlorure de méthacryloyl oxyéthyl triméthylammonium (environ 50/50 en poids) sous forme de dispersion contenant 50 % en poids dudit copolymère dans de l'huile minérale. Cette dispersion est commercialisée sous le nom de "SALCARE SC92" par la Société ALLIED COLLOIDS.

Les copolymères non réticulés de méthacrylamide et de chlorure de méthacryloyloxyéthyl triméthylammonium sont par exemple les produits vendus sous les dénominations commerciales ROHAGIT KF 400 et KF720 par la société ROHM et Haas.

Comme autre dérivés synthétiques, on peut citer les polymères épaississants à chaîne grasses dérivés de cellulose, non ioniques (NATROSOL PLUS de HERCULE) ou cationiques (QUADRISOFT UM 200) ou de polyuréthannes non ioniques (ACULYN 44 ou 46).

Conformément à l'invention, le (ou les) polymère(s) (ii) choisi(s) parmi les polymères fixants, épaississants ou un mélange de ceux-ci est avantageusement présent dans la composition cosmétique dans une quantité comprise entre 0,01 et 20 %, et plus préférentiellement comprise entre 0,1 et 8 % par rapport au poids total de la composition.

### COMPOSITON

Le milieu cosmétiquement acceptable comprend de l'eau et/ou un solvant cosmétiquement acceptable notamment choisi parmi les alcools inférieurs en C₁-C₄, comme l'éthanol, l'isopropanol, le tertio-butanol et le n-butanol ; les polyols, comme le propylèneglycol ; les éthers de polyols; l'acétone et leurs mélanges.

Le milieu cosmétiquement acceptable préféré selon l'invention est un milieu hydroalcoolique, contenant avantageusement de l'eau et de l'etahnol.

La quantité d'eau est comprise entre 15 et 45 % en poids par rapport au poids total de la composition.

De préférence, l'agent propulseur est choisi parmi le diméthyléther, les alcanes en C₃ à C₅, comme le n-butane, le propane, l'isobutane, les hydrocarbures halogénés, comme le le 1,1-difluoroéthane, les mélanges de diméthyléther et d'alcanes en C₃₋₅, les mélanges de 1,1-difluoroethane et de diméthyléther et/ou d'alcanes en C₃₋₅.

Plus préférentiellement, l'agent propulseur est choisi parmi le dimethyléther, les alcanes en C₃ à C₅ et leurs mélanges.

La composition selon l'invention peut comprendre des additifs cosmétiques usuels choisis notamment parmi les agents adhésifs, les agents réducteurs comme les thiols, les corps gras, les adoucissants, les agents anti-mousse, les filtres, les agents antiperspirants, les agents acidifiants, les agents alcalinisants, les colorants, les pigments, les parfums, les conservateurs, les tensioactifs anioniques, cationiques, non ioniques ou amphotères, les polymères non fixants et non épaississants, les silicones volatiles ou non, modifiées ou non, solubles ou insolubles, les huiles végétales, animales, minérales ou de synthèse, les parfums, les protéines et les vitamines, le glycérol et leurs mélanges.

L'homme de métier veillera à choisir les éventuels additifs et leur quantité de manière à ce qu'ils ne nuisent pas aux propriétés des compositions de la présente invention.

Ces additifs sont notamment présents dans la composition selon l'invention en une quantité allant de 0 à 20 % en poids par rapport au poids total de la composition.

L'agent propulseur est notamment présent en une quantité comprise entre 2 et 90 % en poids, de préférence entre 4 et 80 %, mieux encore entre 5 et 65 % en poids par rapport au poids total de la composition.

Les compositions conformes à l'invention sont conditionnées dans un dispositif aérosol usuel en cosmétique. Les compositions pulvérisées peuvent se présenter sous la forme d'un spray ou d'une mousse.

Les compositions conformes à l'invention, pulvérisées à partir du dispositif aérosol, peuvent être utilisées en application rincée ou non, comme compositions de fixation et/ou de maintien des cheveux, compositions de soin de cheveux, shampooings, compositions de conditionnement des cheveux, telles que des compositions destinées à apporter de la douceur aux cheveux ou encore des compositions de maquillage des cheveux.

La présente invention concerne également un procédé de traitement cosmétique des cheveux qui consiste à appliquer une quantité efficace d'une composition telle que décrite ci-dessus, sur les cheveux et à rincer ou non après un éventuel temps de pose.

Selon un mode de réalisation préféré de l'invention, la composition, pulvérisée à partir du dispositif aérosol, peut être utilisée comme produit de coiffage non rincé.

En particulier, les compositions conformes à l'invention sont utilisées pour la mise en forme et/ou le maintien de la chevelure.

Les exemples suivants illustrent la présente invention et ne doivent être considérés en aucune manière comme limitant l'invention.

### Exemples

On a préparé trois produits de coiffage à partir des ingrédients suivants, les pourcentages étant exprimés en poids.

### Exemple 1 (de référence): mousse aérosol

| | |
|---|---|
| Laponite XLG (Laporte) | 0.8% |
| Tégo-bétaïne HS (Goldschmidt) | 0.4% |
| Viscophobe DB 1000 (Amerchol) | 0.86% |
| Conservateurs | qs |
| AEROGAZ 3.2 (1) | 6% |
| Eau | qsp 100% |

| | |
|---|---|
| (1) mélange d'isobutane, de propane et de butane, commercialisé par ATOCHEM | |

### Exemple 2: spray aérosol

| | |
|---|---|
| Laponite XLS (Laporte) | 0.3% |
| Ethanol | 20% |
| Eau | 19.7% |
| DME | 60% |

### Exemple 3 (de référence): spray aérosol

| | |
|---|---|
| Laponite XLS (Laporte) | 0.4% |
| Luviset Si Pur (BASF) | 10% |
| Ethanol | 19% |
| Eau | 10.6% |
| DME | 60% |

Ces produits sont appliqués de préférence sur cheveux humides ou secs. Après mise en forme, on observe un bon effet coiffant sans inconvénient cosmétique.

## Revendications

1. Composition cosmétique, notamment capillaire, conditionnée dans un dispositif aérosol contenant un agent propulseur et une phase liquide qui comprend, dans un milieu cosmétiquement acceptable :
(i) des particules solides contenant au moins 10 % en poids d'au moins un silicate,
(ii) au moins un polymère choisi parmi les polymères fixants, les polymères épaississants ou un mélange de ceux-ci ; et
(iii) de l'eau, dans une quantité comprise entre 15 et 45% en poids par rapport au poids total de la composition,
lesdites particules solides contenant moins de 1 % en poids d'aluminium.

2. Composition selon la revendication 1, **caractérisée par le fait que** le silicate est choisi parmi les silicates de sodium, de magnésium et/ou de lithium.

3. Composition selon l'une quelconque des revendications précédentes, **caractérisée par le fait que** les particules solides présentent une taille primaire moyenne en nombre comprise entre 2 nm et 1 µm.

4. Composition selon l'une quelconque des revendications précédentes, **caractérisée en ce que** les particules contenant au moins 10 % en poids d'au moins un silicate présentent une taille primaire moyenne en nombre comprise entre 5 et 500 nm, et plus préférentiellement entre 10 et 250 nm.

5. Composition selon l'une quelconque des revendications précédentes, **caractérisée en ce que** les particules contiennent au moins 50 % en poids de silicate, de préférence 70 % en poids, et plus préférentiellement encore, plus de 90 % en poids de silicate.

6. Composition selon l'une quelconque des revendications précédentes, **caractérisée en ce que** les particules contenant du silicate sont présentes en une quantité comprise entre 0,01 % et 10 % en poids, et de préférence entre 0,5 % et 3 % en poids par rapport au poids total de la composition.

7. Composition selon l'une quelconque des revendications précédentes, **caractérisée en ce que** les polymères fixants sont choisis parmi les polymères anioniques et non ioniques.

8. Composition selon la revendication 7, **caractérisée par le fait que** le polymère anionique est choisi parmi :
- les polymères comportant des motifs carboxyliques dérivant de monomères mono ou diacides carboxyliques insaturés de formule : 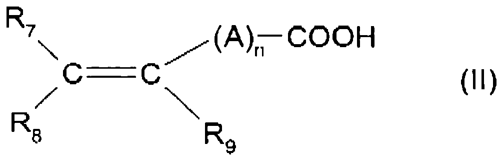 dans laquelle n est un nombre entier de 0 à 10, A désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle ;
- les polymères comprenant des motifs dérivant d'acide sulfonique tels que des motifs vinylsulfonique, styrènesulfonique, acrylamido alkylsulfonique, dans la formule précitée, un radical alkyle inférieur désigne un groupement ayant 1 à 4 atomes de carbone

9. Composition selon la revendication 7, **caractérisée par le fait que** le polymère non ionique est choisi parmi :
- les homopolymères ou copolymères de vinylpyrrolidone ou de vinylcaprolactame ;
- les polyalkyloxazolines;
- les homopolymères d'acétate de vinyle;
- les copolymères d'acétate de vinyle et d'ester acrylique;
- les copolymères d'acétate de vinyle et d'éthylène;
- les copolymères d'acétate de vinyle et d'ester maléïque;
- les copolymères de polyéthylène et d'anhydride maléïque;
- les homopolymères d'acrylates d'alkyle et les homopolymères de méthacrylates d'alkyle;
- les copolymères d'esters acryliques tels que par exemple les copolymères d'acrylates d'alkyle et de méthacrylates d'alkyle ;
- les copolymères d'acrylonitrile et d'un monomère non ionique choisi par exemple parmi le butadiène et les (méth)acrylates d'alkyle;
- les copolymères d'acrylate d'alkyle et d'uréthanne ; et
- les polyamides.

10. Composition selon l'une quelconque des revendications 1 à 6, **caractérisée par le fait que** l'épaississant est choisi parmi les copolymères d'acide acrylique ou méthacrylique comprenant au moins un motif acrylate d'alkyle en C1 à C30 et/ou un motif uréthanne éventuellement substitué par une chaîne grasse.

11. Composition selon l'une quelconque des revendications 1 à 6, **caractérisée par le fait que** l'épaississant est choisi parmi les polymères ou copolymères d'acide acrylique et/ou méthacrylique, comme les copolymères acide acrylique/acrylate d'éthyle et les polymères carboxyvinyliques.

12. Composition selon l'une quelconque des revendications 1 à 6, **caractérisée par le fait que** le polymère fixant est choisi parmi :
- les dérivés de cellulose cationique;
- les gommes de guar contenant des groupements cationiques trialkylammonium.

13. Composition selon l'une quelconque des revendications 1 à 6, **caractérisée par le fait que** le polymère (ii) est présent dans la composition cosmétique dans une proportion comprise entre 0,01 et 8 %, et de préférence comprise entre 0,1 et 8 % par rapport au poids total de la composition.

14. Composition selon l'une quelconque des revendications précédentes, **caractérisée en ce que** l'agent propulseur est choisi parmi le diméthyléther, les alcanes en C₃ à C₅, comme le n-butane, le propane, l'isobutane, les hydrocarbures halogénés, comme le le 1,1-difluoroéthane, les mélanges de diméthyléther et d'alcanes en C₃₋₅, les mélanges de 1,1-difluoroéthane et de diméthyléther et/ou d'alcanes en C₃₋₅.

15. Composition selon la revendication précédente, **caractérisée en ce que** l'agent propulseur est choisi parmi le diméthyléther et les alcanes en C₃ à C₅ et leurs mélanges.

16. Composition selon l'une quelconque des revendications précédentes, **caractérisée en ce que** l'agent propulseur est présent en une quantité comprise entre 2 et 90 % en poids, de préférence entre 4 et 80 %, mieux encore entre 5 et 65 % en poids par rapport au poids total de la composition.

17. Composition selon l'une quelconque des revendications précédentes, **caractérisée en ce qu'**elle comprend des additifs cosmétiques usuels choisis parmi les agents adhésifs, les agents réducteurs comme les thiols, les corps gras, les adoucissants, les agents anti-mousse, les filtres, les agents antiperspirants, les agents acidifiants, les agents alcalinisants, les colorants, les pigments, les parfums, les conservateurs, les tensioactifs anioniques, cationiques, non ioniques ou amphotères, les polymères non fixants et non épaississants, les silicones volatiles ou non, modifiées ou non, solubles ou insolubles, les huiles végétales, animales, minérales ou de synthèse, les parfums, les protéines et les vitamines, le glycérol et leurs mélanges.

18. Procédé de traitement cosmétique des cheveux mettant en oeuvre une composition selon l'une quelconque des revendications 1 à 17.

19. Utilisation d'une composition selon l'une quelconque des revendications 1 à 17 en cosmétique capillaire, notamment pour la mise en forme et/ou le maintien de la chevelure.

## Claims

1. Cosmetic composition, notably for the hair, packaged in an aerosol device containing a propellant and a liquid phase which comprises, in a cosmetically acceptable medium:
(i) solid particles containing at least 10% by weight of at least one silicate,
(ii) at least one polymer selected from fixing polymers, thickening polymers or a mixture thereof, and
(iii) water, in a quantity of between 15 and 45% by weight in relation to the total weight of the composition;
said solid particles containing less than 1% by weight of aluminum.

2. Composition according to Claim 1, **characterized in that** the silicate is selected from sodium, magnesium and/or lithium silicates.

3. Composition according to either one of the preceding claims, **characterized in that** the solid particles have a number-average primary size of between 2 nm and 1 µm.

4. Composition according to any one of the preceding claims, **characterized in that** the particles containing at least 10% by weight of at least one silicate have a number-average primary size of between 5 and 500 nm, and more preferentially between 10 and 250 nm.

5. Composition according to any one of the preceding claims, **characterized in that** the particles contain at least 50% by weight of silicate, preferably 70% by weight, and more preferentially still, more than 90% by weight of silicate.

6. Composition according to any one of the preceding claims, **characterized in that** the particles containing silicate are present in a quantity of between 0.01% and 10% by weight, and preferably between 0.5% and 3% by weight in relation to the total weight of the composition.

7. Composition according to any one of the preceding claims, **characterized in that** the fixing polymers are selected from anionic and nonionic polymers.

8. Composition according to Claim 7, **characterized in that** the anionic polymer is selected from:
- polymers comprising carboxylic units deriving from unsaturated carboxylic mono- or diacid monomers of formula: 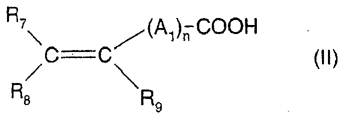 in which n is a whole number from 0 to 10, A denotes a methylene group, possibly linked to the carbon atom of the unsaturated group or to the neighboring methylene group when n is greater than 1 by means of a heteroatom such as oxygen or sulfur, R₇ denotes a hydrogen atom, a phenyl or benzyl group, R₈ denotes a hydrogen atom, a lower alkyl or carboxyl group, R₉ denotes a hydrogen atom, a lower alkyl group, a -CH₂-COOH, phenyl or benzyl group;
- polymers comprising units deriving from sulfonic acid such as vinylsulfonic, styrenesulfonic, acrylamidoalkylsulfonic units;
in the above formula, a lower alkyl radical denotes a group having 1 to 4 carbon atoms.

9. Composition according to Claim 7, **characterized in that** the nonionic polymer is selected from:
- the homopolymers or copolymers of vinylpyrrolidone or vinylcaprolactam;
- polyalkyloxazolines;
- the homopolymers of vinyl acetate;
- the copolymers of vinyl acetate and acrylic ester;
- the copolymers of vinyl acetate and ethylene;
- the copolymers of vinyl acetate and maleic ester;
- the copolymers of polyethylene and maleic anhydride;
- the homopolymers of alkyl acrylates and the homopolymers of alkyl methacrylates;
- the copolymers of acrylic esters such as for example the copolymers of alkyl acrylates and alkyl methacrylates;
- the copolymers of acrylonitrile and a nonionic monomer selected for example from butadiene and the alkyl (meth)acrylates;
- the copolymers of alkyl acrylate and urethane; and
- polyamides.

10. Composition according to any one of Claims 1 to 6, **characterized in that** the thickener is selected from copolymers of acrylic or methacrylic acid comprising at least one C1 to C30 alkyl acrylate unit and/or one urethane unit possibly substituted by a fatty chain.

11. Composition according to any one of Claims 1 to 6, **characterized in that** the thickener is selected from polymers or copolymers of acrylic and/or methacrylic acid, such as acrylic acid/ethyl acrylate copolymers and carboxyvinyl polymers.

12. Composition according to any one of Claims 1 to 6, **characterized in that** the fixing polymer is selected from:
- derivatives of cationic cellulose;
- guar gums containing cationic trialkylammonium groups.

13. Composition according to any one of Claims 1 to 6, **characterized in that** polymer (ii) is present in the cosmetic composition in a proportion of between 0.01 and 8%, and preferably of between 0.1 and 8% in relation to the total weight of the composition.

14. Composition according to any one of the preceding claims, **characterized in that** the propellant is selected from dimethylether, C₃ to C₅ alkanes, such as n-butane, propane, isobutane, halogenated hydrocarbons, such as 1,1-difluoroethane, mixtures of dimethylether and C₃₋₅ alkanes, mixtures of 1,1-difluoroethane and dimethylether and/or C₃₋₅ alkanes.

15. Composition according to the preceding claim, **characterized in that** the propellant is selected from dimethylether and C₃ to C₅ alkanes and their mixtures.

16. Composition according to any one of the preceding claims, **characterized in that** the propellant is present in a quantity of between 2 and 90% by weight, preferably between 4 and 80%, better still between 5 and 65% by weight in relation to the total weight of the composition.

17. Composition according to any one of the preceding claims, **characterized in that** it comprises usual cosmetic additives selected from adhesive agents, reducing agents such as thiols, fatty substances, softeners, defoamers, filters, antiperspirants, acidifying agents, alkalinizing agents, colorants, pigments, perfumes, preservatives, anionic, cationic, nonionic or amphoteric surfactants, non-fixing and non-thickening polymers, silicones volatile or not, modified or not, soluble or insoluble, vegetable, animal, mineral or synthetic oils, perfumes, proteins and vitamins, glycerol and their mixtures.

18. Cosmetic hair treatment process using a composition according to any one of Claims 1 to 17.

19. Use of a composition according to any one of Claims 1 to 17 in hair cosmetics, notably for forming and/or maintaining the head of hair.

## Patentansprüche

1. Kosmetikzusammensetzung, insbesondere Haarzusammensetzung, die in einem Aerosolbehälter verpackt ist, der ein Treibmittel und eine flüssige Phase, die in einem kosmetisch unbedenklichen Medium Folgendes umfasst:
(i) feste Partikel, die mindestens 10 Gew.-% an mindestens einem Silikat enthalten,
(ii) mindestens ein Polymer aus der Reihe der fixierenden Polymere, der verdickenden Polymere oder eine Mischung dieser Polymere sowie
(iii) Wasser in einer Menge zwischen 15 und 45 Gew.-% in Bezug auf das Gesamtgewicht der Zusammensetzung,
wobei die festen Partikel mindestens 1 Gew.-% Aluminium enthalten, enthält.

2. Zusammensetzung nach Anspruch 1, **dadurch gekennzeichnet, dass** das Silikat aus der Reihe der Natrium-, Magnesium- und/oder Lithiumsilikate stammt.

3. Zusammensetzung nach einem der vorhergehenden Ansprüche, **dadurch gekennzeichnet, dass** die festen Partikel eine zahlenmittlere Primärgröße zwischen 2 nm und 1 µm aufweisen.

4. Zusammensetzung nach einem der vorhergehenden Ansprüche, **dadurch gekennzeichnet, dass** die Partikel, die mindestens 10 Gew.-% an mindestens einem Silikat enthalten, eine zahlenmittlere Primärgröße zwischen 5 und 500 nm, stärker bevorzugt zwischen 10 und 250 nm aufweisen.

5. Zusammensetzung nach einem der vorhergehenden Ansprüche, **dadurch gekennzeichnet, dass** die Partikel mindestens 50 Gew.-% Silikat, vorzugsweise 70 Gew.-% und noch stärker bevorzugt mehr als 90 Gew.-% Silikat enthalten.

6. Zusammensetzung nach einem der vorhergehenden Ansprüche, **dadurch gekennzeichnet, dass** die silikathaltigen Teilchen in einer Menge von zwischen 0,01 Gew.-% und 10 Gew.-%, vorzugweise zwischen 0,5 Gew.-% und 3 Gew.-%, in Bezug auf das Gesamtgewicht der Zusammensetzung vorliegen.

7. Zusammensetzung nach einem der vorhergehenden Ansprüche, **dadurch gekennzeichnet, dass** die fixierenden Polymere aus der Reihe der anionischen Polymere und der nichtionischen Polymere stammen.

8. Zusammensetzung nach Anspruch 7, **dadurch gekennzeichnet, dass** das anionische Polymer aus der folgenden Reihe stammt:
- Polymere mit Carbonsäureeinheiten, die sich von ungesättigten Mono- oder Dicarbonsäuremonomeren der folgenden Formel ableiten: 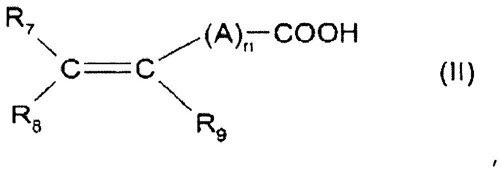 in der n eine ganze Zahl von 0 bis 10 bedeutet, A eine Methylengruppe, die gegebenenfalls an das Kohlenstoffatom der ungesättigten Gruppe oder an die benachbarte Methylengruppe, wenn n größer als 1 ist, gebunden ist, und zwar über ein Heteroatom wie Sauerstoff oder Schwefel, R₇ ein Wasserstoffatom, eine Phenylgruppe oder eine Benzylgruppe bedeutet, R₈ ein Wasserstoffatom, eine Niederalkylgruppe oder eine Carboxylgruppe bedeutet, R₉ ein Wasserstoffatom, eine Niederalkylgruppe, eine -CH₂-COOH-Gruppe, eine Phenyl- oder eine Benzylgruppe bedeutet;
- Polymere mit Einheiten, die sich von Sulfonsäure ableiten, wie Vinylsulfonsäureeinheiten, Styrolsulfonsäureeinheiten, Acrylamidoalkylsulfonsäureeinheiten,
wobei in der oben genannten Formel ein Niederalkylrest eine Gruppierung mit 1 bis 4 Kohlenstoffatomen bedeutet.

9. Zusammensetzung nach Anspruch 7, **dadurch gekennzeichnet, dass** das nichtionische Polymer aus der folgenden Reihe stammt:
- Vinylpyrrolidon- oder Vinylcaprolactamhomopolymere oder -copolymere;
- Polyalkyloxazoline;
- Vinylacetathomopolymere;
- Vinylacetat-Acrylsäureester-Copolymere;
- Vinylacetat-Ethylen-Copolymere;
- Vinylacetat-Maleinsäure-Copolymere;
- Polyethylen-Maleinsäureanhydrid-Copolymere;
- Alkylacrylathomopolymere und Alkylmethacrylathomopolymere;
- Acrylsäureestercopolymere, wie zum Beispiel die Alkylacrylatcopolymere und die Alkylmethacrylatcopolymere;
- Copolymere von Acrylnitril und einem nichtionischen Monomer, das zum Beispiel aus der Reihe Butadien und Alkyl(meth)acrylate ausgewählt ist;
- Alkylacrylat-Urethan-Copolymere sowie
- Polymamide.

10. Zusammensetzung nach einem der Ansprüche 1 bis 6, **dadurch gekennzeichnet, dass** das Verdickungsmittel aus der Reihe der Acrylsäure- oder Methacrylsäurecopolymere mit mindestens einer C1- bis C30-Alkylacrylateinheit und/oder einer gegebenenfalls durch eine Fettkette substituierten Urethaneinheit stammt.

11. Zusammensetzung nach einem der Ansprüche 1 bis 6, **dadurch gekennzeichnet, dass** das Verdickungsmittel aus der Reihe der Acrylsäure- und/oder Methacrylsäurepolymere oder -copolymere, wie der Acrylsäure-Ethylacrylat-Copolymere, und der Carboxyvinylpolymere stammt.

12. Zusammensetzung nach einem der Ansprüche 1 bis 6, **dadurch gekennzeichnet, dass** das fixierende Polymer aus der Reihe
- der kationischen Cellulosederivate;
- der Guargummen mit kationischen Trialkylammoniumgruppen stammt.

13. Zusammensetzung nach einem der Ansprüche 1 bis 6, **dadurch gekennzeichnet, dass** das Polymer (ii) in der Kosmetikzusammensetzung in einem Anteil von zwischen 0,01 und 8 %, vorzugweise zwischen 0,1 und 8%, in Bezug auf das Gesamtgewicht der Zusammensetzung vorliegt.

14. Zusammensetzung nach einem der vorhergehenden Ansprüche, **dadurch gekennzeichnet, dass** das Treibmittel aus der Reihe Dimethylether, C₃- bis C₅-Alkane wie n-Butan, Propan, Isobutan, halogenierte Kohlenwasserstoffe wie 1,1-Difluorethan, Mischungen aus Dimethylether und C₃₋₅-Alkanen, Mischungen von 1,1-Difluorethan und Dimethylether und/oder C₃₋₅-Alkanen stammt.

15. Zusammensetzung nach dem vorhergehenden Anspruch, **dadurch gekennzeichnet, dass** das Treibmittel aus der Reihe Dimethylether und C₃- bis C₅-Alkane und ihren Mischungen stammt.

16. Zusammensetzung nach einem der vorhergehenden Ansprüche, **dadurch gekennzeichnet, dass** das Treibmittel in einer Menge von zwischen 2 und 90 Gew.-%, vorzugweise zwischen 4 und 80 Gew.-%, noch stärker bevorzugt zwischen 5 und 65 Gew.-%, in Bezug auf das Gesamtgewicht der Zusammensetzung vorliegt.

17. Zusammensetzung nach einem der vorhergehenden Ansprüche, **dadurch gekennzeichnet, dass** sie übliche Kosmetikzusatzstoffe aus der Reihe Haftmittel, Reduktionsmittel wie Thiole, Fettkörper, Weichmacher, Entschäumer, Filter, Antiperspirantien, Säuerungsmittel, Alkalinisierungsmittel, Farbstoffe, Pigmente, Duftstoffe, Konservierungsmittel, anionische, kationische, nichtionische oder amphotere Tenside, nichtfixierende und nichtverdickende Polymere, lösliche oder unlösliche, modifizierte oder unmodifizierte, flüchtige oder nichtflüchtige Silikone, pflanzliche Öle, tierische Öle, Mineralöle oder Syntheseöle, Duftstoffe, Proteine und Vitamine, Glyzerin und Mischungen davon umfasst.

18. Verfahren für die kosmetische Behandlung des Haars, bei der eine Zusammensetzung nach einem der Ansprüche 1 bis 17 eingesetzt wird.

19. Verwendung einer Zusammensetzung nach einem der Ansprüche 1 bis 17 in der Haarkosmetik, insbesondere um das Haar zu formen und/oder ihm Halt zu geben.
